# EUROPÄISCHE PATENTANMELDUNG

(11) **EP 3 453 700 A1**
(43) Veröffentlichungstag der Anmeldung: **13.03.2019**
(21) Anmeldenummer: 17189560.0
(22) Anmeldetag: 06.09.2017
(51) Int. Cl.: C07C 263/10, C07C 265/14, B01J 8/02, C01B 32/80, C08G 64/24

(54) **VERFAHREN ZUR REINIGUNG PHOSGEN-FÜHRENDER APPARATE**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die Erfindung betrifft ein Verfahren zur Reinigung eines Phosgen-führenden Apparats, umfassend eine Spülung des Apparats mit heißem Inertgas, gefolgt von einer Spülung des Apparats mit kaltem Inertgas, Durchleiten von gasförmigem Ammoniak zur Zersetzung von Phosgenresten unter kontinuierlichem Temperaturanstieg, bis ein Temperaturmaximum im Bereich von 30 °C bis 120 °C erreicht wird, nach Erreichung des Temperaturmaximums, Abstellen der Ammoniakzufuhr und Durchleiten von Inertgas, optional (und bevorzugt) gefolgt von einer Spülung des Apparats mit einem wässrigem Strom.

## Beschreibung

Die Erfindung betrifft ein Verfahren zur Reinigung eines Phosgen-führenden Apparats, umfassend eine Spülung des Apparats mit heißem Inertgas, gefolgt von einer Spülung des Apparats mit kaltem Inertgas, Durchleiten von gasförmigem Ammoniak zur Zersetzung von Phosgenresten unter kontinuierlichem Temperaturanstieg, bis ein Temperaturmaximum im Bereich von 30 °C bis 120 °C erreicht wird, nach Erreichung des Temperaturmaximums, Abstellen der Ammoniakzufuhr und Durchleiten von Inertgas, optional (und bevorzugt) gefolgt von einer Spülung des Apparats mit einem wässrigem Strom.

Phosgen findet in vielen Bereichen der Chemie Anwendung, sei es als Hilfsstoff oder als Zwischenprodukt. Das mengenmäßig größte Einsatzgebiet stellt die Herstellung von Di- und/oder Polyisocyanaten als Ausgangsstoffe für die Polyurethanchemie dar. Insbesondere sind hier Toluylendiisocyanat (insbesondere *meta*-Toluylendiisocyanat), Naphthalindiisocyanat (insbesondere 1,5-Naphthalindiisocyanat), 1,5-Pentandiisocyanat, 1,6-Hexamethylendiisocyanat, Isophorondiisocyanat, Diisocyanatodicyclohexylmethan, Xylylendiisocyanat (insbesondere 1,3- und/oder 1,4-Xylylendiisocyanat) sowie die Di- und Polyamine der Diphenylmethanreihe zu nennen. Ein weiteres wichtiges Einsatzgebiet für die Verwendung von Phosgen ist die Herstellung von Polycarbonaten.

Die Produktionsanlagen zur Herstellung solcher Stoffe umfassen naturgemäß zahlreiche Phosgen-führende Apparate, beispielsweise Reaktoren zur Phosgenherstellung oder Reaktoren zur Umsetzung von Phosgen mit entsprechenden reaktiven Ausgangsmaterialien wie Aminen oder Phenolen. Des Weiteren sind zahlreiche Aufarbeitungsapparate wie Destillationskolonnen als Phosgen-führende Apparate anzusehen. Gleiches gilt selbstredend auch für zahlreiche Peripheriegeräte wie Tanks und Rohrleitungen.

In allen Fällen kann es erforderlich werden, solche Apparate zu öffnen, beispielsweise für Wartungs-, Instandsetzungs- und Reinigungsarbeiten oder um beispielsweise verbrauchten Katalysator aus einem Reaktor zu entfernen. Es ist gehört eingedenk der hohen Giftigkeit von Phosgen schon seit vielen Jahrzehnten zur guten Industriepraxis, solche Apparate vor dem Öffnen sehr sorgfältig von Phosgen zu befreien. In der Industrie gebräuchliche Anleitungen hierzu sind dem Fachmann bekannt. Beispielhaft seien die *"*Phosgen Safety Practices for design, production and processing" des International Isocyanate Institute Inc. genannt. In der Ausgabe von 2012 wird dort in Teil 2 unter Ziffer 3.2.3 eine Prozedur beschrieben, die unter anderem das Spülen des Apparats mit Stickstoff, das Spülen des Apparats mit Ammoniak und das Fluten des Apparats mit Wasser, Natronlauge oder Ammoniakwasser beinhaltet. Ebenfalls hingewiesen seien auf die *"*Phosgene Safe Practice Guidelines" des American Chemical Council. Dort wird in der Fassung des Jahres 2014 in Kapitel 9.0 ("Equipment Cleaning and Repair*"*) offenbart, dass sich schwierig zu reinigende Apparate unter anderem mit wasserfreiem Ammoniak von Phosgen befreien lassen.

Die Herstellung von Phosgen erfolgt üblicherweise durch Reaktion von Kohlenstoffmonoxid und Chlor an einem Aktivkohlekatalysator. Bevor ein dazu eingesetzter Reaktor geöffnet werden kann, muss sichergestellt werden, dass auch die darin enthaltende Aktivkohle von Phosgen befreit ist, was aufgrund deren großer innerer Oberfläche sehr sorgfältig durchgeführt werden muss. Mit dieser Problemstellung befasst sich die internationale Patentanmeldung WO 2016/109987 A1, die auf ein Verfahren zur raschen Befreiung einer Phosgenherstellungskolonne von Phosgen gerichtet ist. Dieses Verfahren umfasst nach Abstellen der Zufuhr der Ausgangsmaterialien Kohlenstoffmonoxid und Chlor zunächst einen Schritt A) des Spülens mit Stickstoff, um die Hauptmenge an Phosgen aus dem Reaktor auszutreiben. Es folgt optional ein Schritt B), in dem Ammoniakgas in die Phosgenherstellungskolonne (deren Auslassöffnung nun verschlossenen ist) geleitet wird, bis sich ein Druck im Bereich von 0,11 MPa bis 5 MPa einstellt. Nach 1 Stunde bis 10 Stunden wird das System durch Öffnen der Auslassöffnung der Phosgenherstellungskolonne entspannt. Dieser Vorgang des Aufdrückens von Ammoniak und Entspannens wird erforderlichenfalls mehrfach wiederholt. Hierdurch soll zum Einen die Verstopfung der nachgelagerten Verrohrung verhindert und zum Anderen erreicht werden, dass auch in den Poren des Aktivkohlekatalysators befindliches Phosgen abreagiert. Diesem optionalen Schritt B) schließt sich ein Schritt C) des Durchleitens von Ammoniakgas durch die Phosgenherstellungskolonne an. Dieser Schritt wird so lange durchgeführt, bis der im Auslass der Phosgenherstellungskolonne gemessene Phosgengehalt auf einen Wert von höchstens 1 ppm fällt. Der Erfolg der Phosgenzersetzung wird also demnach nur durch die *Analyse des Restphosgengehaltes* überprüft. Die Anmeldung offenbart ferner im Falle des Einsatzes eines Rohrbündelreaktors zur Phosgenherstellung die Reinigung des Reaktors mit Wasser nach Entnahme der Aktivkohle.

Die Überprüfung des Erfolgs der Phosgenzersetzung allein über Probennahmen und Analyse des Restphosgengehalts bei Zersetzung des Phosgens nur mit gasförmigen Ammoniak ist jedoch nicht unkritisch, da nicht auszuschließen ist, dass verborgene "Phosgennester" hiermit übersehen werden. Solche "Phosgennester" können insbesondere in Rohrbündelreaktoren zur Phosgenherstellung auftreten, wenn Rohre durch Zusammenbacken von Katalysatorpartikeln verstopfen.

Es bestand daher ein Bedarf an weiteren Verbesserungen auf dem Gebiet der Reinigung, d. h. insbesondere der Befreiung von Phosgen, Phosgen-führender Apparate. Es wäre insbesondere wünschenswert, auf einfache Art und Weise feststellen zu können, wann der Reinigungsvorgang, d. h. insbesondere die Zersetzung von restlichem Phosgen, abgeschlossen ist und der Apparat gefahrlos geöffnet werden kann. Insbesondere wäre es wünschenswert, die an sich attraktive Phosgenzersetzung mit gasförmigem Ammoniak weiter zu verbessern.

Diesem Bedarf Rechnung tragend ist ein Gegenstand der vorliegenden Erfindung ein Verfahren zur Reinigung eines Phosgen-führenden Apparats, umfassend die Schritte:
a) Bereitstellung einer Einrichtung zur Gasabfuhr aus dem zu reinigenden Apparat und einer Einrichtung zur Gaszufuhr in den zu reinigenden Apparat und Absperren aller gegebenenfalls darüber hinaus vorhandenen mit dem zu reinigenden Apparat verbundenen Leitungen;
b) Durchleiten eines Inertgases, insbesondere Durchleiten von Stickstoff, einer Temperatur im Bereich von 100 °C bis 500 °C, bevorzugt im Bereich von 120 °C bis 400 °C, besonders bevorzugt im Bereich von 150 °C bis 350 °C, ganz besonders bevorzugt im Bereich von 250 °C bis 300 °C, durch den zu reinigenden Apparat, und zwar insbesondere bei einem Druck in dem zu reinigenden Apparat unterhalb von Umgebungsdruck, bevorzugt bei einem Druck im Bereich von 10 mbar bis 1000 mbar, besonders bevorzugt im Bereich von 100 mbar bis 990 mbar, ganz besonders bevorzugt im Bereich von 500 mbar bis 980 mbar,
   wobei ferner Schritt b) insbesondere so lange durchgeführt wird, bis der kontinuierlich oder in Intervallen von bis zu 300 h, bevorzugt in Intervallen im Bereich von 50 h bis 300 h, besonders bevorzugt von 60 h bis 200 h, ganz besonders bevorzugt von 70 h bis 150 h und außerordentlich ganz besonders bevorzugt von 80 h bis 120 h, in dem aus dem zu reinigenden Apparat abgeführten Inertgas per Farbumschlag infolge der Reaktion von Phosgen mit Ethylanilin und Dimethylaminobenzaldehyd im Temperaturbereich von 10 °C bis 30 °C bestimmte, auf das Gesamtvolumen des die mindestens eine Ableitung pro Zeitspanne durchströmenden Gasstroms bezogene, Volumenanteil an Phosgen auf einen Wert von kleiner oder gleich 1 ppm fällt;
c) Abstellen des (heißen) Inertgasstroms aus Schritt b), gefolgt von Abkühlen des zu reinigenden Apparates auf eine zuvor festgelegte, in der Einrichtung zur Gasabfuhr gemessene, Temperatur im Bereich von 0 °C bis 70 °C, bevorzugt im Bereich von 10 °C bis 60 °C, besonders bevorzugt im Bereich von 20 °C bis 50 °C, ganz besonders bevorzugt im Bereich von 30 °C bis 40 °C, durch Durchleiten eines Inertgases, insbesondere Stickstoff, einer Temperatur gleich oder unterhalb der zuvor festgelegten Temperatur,
   wobei ferner der Inertgasstrom aus Schritt c) auch nach erfolgter Abkühlung des zu reinigenden Apparates weiter aufrecht erhalten werden kann;
d) sobald das Abkühlen des zu reinigenden Apparates in Schritt c) erfolgt ist, Durchleiten von gasförmigem Ammoniak aus einer Ammoniakgasquelle, insbesondere durch Einspeisung in den aufrecht erhaltenen Intertgasstrom aus Schritt c), unter Erhalt eines Ammoniak-haltigen Gasgemisches, wobei der Mengenstrom des gasförmigen Ammoniaks so gewählt wird, dass die Temperatur in dem aus dem zu reinigenden Apparat abgeführten Gasgemisch kontinuierlich ansteigt und nach Erreichen eines Maximums wieder abfällt, wobei das Maximum der Temperatur im Bereich von 30 °C bis 120 °C, bevorzugt im Bereich von 50 °C bis 110 °C, besonders bevorzugt im Bereich von 60 °C bis 100 °C und ganz besonders bevorzugt im Bereich von 70 °C bis 90 °C, liegt;
e) frühestens nach Erreichen des Temperaturmaximums in Schritt d), Abtrennen der Ammoniakgasquelle von dem zu reinigenden Apparat, gefolgt von Durchleiten eines Intergases durch den zu reinigenden Apparat, insbesondere durch weitere Aufrechterhaltung des Inertgasstromes aus Schritt c);
f) optional, nach Abstellung des Inertgasstromes, Bereitstellung einer Flüssigkeitszuleitung in den und einer Flüssigkeitsableitung aus dem zu reinigenden Apparat und Durchleiten eines wässrigen Stroms, insbesondere Wasser, durch den zu reinigenden Apparat, insbesondere mindestens bis der pH-Wert des aus dem Apparat austretenden wässrigen Stroms im Bereich von 7,0 bis 10,0, bevorzugt von 7,0 bis 9,0, besonders bevorzugt von 7,0 bis 8,0, liegt.

*"Phosgen-führende Apparate"* im Sinne der Erfindung sind alle Anlagenteile, die im Betrieb mit Phosgen in Kontakt treten können, also insbesondere Reaktoren zur Phosgenherstellung, Reaktoren zur Umsetzung von Phosgen mit einem reaktionsfähigen Ausgangsmaterial, Aufarbeitungsapparate zur Reinigung von Produkten, die unter Einsatz von Phosgen hergestellt wurden (insbesondere Destillationskolonnen) sowie Peripheriegeräte wie Rohrleitungen und Behälter (z. B. Tankbehälter).

### Es folgt zunächst eine Kurzzusammenfassung verschiedener möglicher Ausführungsformen der Erfindung:

In einer **ersten Ausführungsform** der Erfindung, die mit allen anderen Ausführungsformen kombiniert werden kann, wird Schritt b) bei einem Druck unterhalb von Umgebungsdruck durchgeführt.

In einer **zweiten Ausführungsform** der Erfindung, die eine besondere Ausgestaltung der ersten Ausführungsform ist, liegt der Druck in Schritt b) im Bereich von 10 mbar bis 1000 mbar, bevorzugt im Bereich von 100 mbar bis 990 mbar, besonders bevorzugt im Bereich von 500 mbar bis 980 mbar.

In einer **dritten Ausführungsform** der Erfindung, die mit allen anderen Ausführungsformen kombiniert werden kann, wird Schritt b) so lange durchgeführt, bis der kontinuierlich oder in Intervallen von bis zu 300 h, bevorzugt in Intervallen im Bereich von 50 h bis 300 h, besonders bevorzugt von 60 h bis 200 h, ganz besonders bevorzugt von 70 h bis 150 h und außerordentlich ganz besonders bevorzugt von 80 h bis 120 h, in dem aus dem zu reinigenden Apparat abgeführten Inertgas per Farbumschlag infolge der Reaktion von Phosgen mit Ethylanilin und Dimethylaminobenzaldehyd im Temperaturbereich von 10 °C bis 30 °C bestimmte, auf das Gesamtvolumen des die mindestens eine Ableitung pro Zeitspanne durchströmenden Gasstroms bezogene, Volumenanteil an Phosgen auf einen Wert von kleiner oder gleich 1 ppm fällt.

In einer **vierten Ausführungsform** der Erfindung, die mit allen anderen Ausführungsformen kombiniert werden kann, liegt die zuvor festgelegte, in der Einrichtung zur Gasabfuhr gemessene, Temperatur aus Schritt c) im Bereich von 10 °C bis 60 °C, bevorzugt im Bereich von 20 °C bis 50 °C, besonders bevorzugt im Bereich von 30 °C bis 40 °C.

In einer **fünften Ausführungsform** der Erfindung, die mit allen anderen Ausführungsformen kombiniert werden kann, wird der Inertgasstrom aus Schritt c) nach erfolgter Abkühlung des zu reinigenden Apparates weiter aufrecht erhalten.

In einer **sechsten Ausführungsform** der Erfindung, die eine besondere Ausgestaltung der fünften Ausführungsform ist, erfolgt das Durchleiten von gasförmigem Ammoniak aus einer Ammoniakgasquelle in Schritt d) durch Einspeisung in den aufrecht erhaltenen Intertgasstrom aus Schritt c).

In einer **siebten Ausführungsform** der Erfindung, die eine besondere Ausgestaltung der fünften und sechsten Ausführungsform ist, wird das Durchleiten eines Intergases durch den zu reinigenden Apparat in Schritt e) nur durch Aufrechterhaltung des Inertgasstromes aus Schritt c) bewirkt.

In einer **achten Ausführungsform** der Erfindung, die mit allen anderen Ausführungsformen kombinierbar ist, wird Schritt f) durchgeführt, und der wässrige Strom wird mindestens so lange durch den zu reinigenden Apparat geleitet, bis der pH-Wert des aus dem Apparat austretenden wässrigen Stroms im Bereich von 7,0 bis 10,0, bevorzugt von 7,0 bis 9,0, besonders bevorzugt von 7,0 bis 8,0, liegt.

In einer **neunten Ausführungsform** der Erfindung, die mit allen anderen Ausführungsformen kombinierbar ist, ist der zu reinigende Apparat (i) ein mit Aktivkohle befüllter Reaktor geeignet zur Herstellung von Phosgen aus Kohlenstoffmonoxid und Chlor oder (ii) ein mit Aktivkohle befüllter Reaktor geeignet zur Absorption oder Zersetzung von Phosgen in Phosgen-haltigen Gasströmen, wobei der Mengenstrom an gasförmigem Ammoniak, der in Schritt d) durch den mit Aktivkohle befüllten Reaktor geleitet wird, im Bereich von 3 L/h bis 30 L/h pro Tonne Aktivkohle liegt.

In einer **zehnten Ausführungsform** der Erfindung, die mit allen anderen Ausführungsformen kombinierbar ist, ist der zu reinigende Apparat ein mit Aktivkohle befüllter Reaktor geeignet zur Herstellung von Phosgen aus Kohlenstoffmonoxid und Chlor ist.

In einer **elften Ausführungsform** der Erfindung, die eine besondere Ausgestaltung der zehnten Ausführungsform ist, ist der mit Aktivkohle befüllte Reaktor geeignet zur Herstellung von Phosgen aus Kohlenstoffmonoxid und Chlor Bestandteil einer Produktionsanlage zur Herstellung eines chemischen Produkts durch Umsetzung eines mit Phosgen reaktionsfähigen Ausgangsmaterials mit Phosgen.

In einer **zwölften Ausführungsform** der Erfindung, die eine besondere Ausgestaltung der zehnten und elften Ausführungsform ist, ist das mit Phosgen reaktionsfähige Ausgangsmaterial eine Verbindung mit zwei oder mehr phenolischen Hydroxygruppen oder eine Verbindung mit zwei oder mehr primären Amingruppen.

In einer **dreizehnten Ausführungsform** der Erfindung, die eine besondere Ausgestaltung der elften und zwölften Ausführungsform ist, verfügt die Produktionsanlage zur Herstellung eines chemischen Produkts über *n* unabhängig voneinander regelbare, mit Aktivkohle befüllte Reaktoren geeignet zur Herstellung von Phosgen aus Kohlenstoffmonoxid und Chlor, wobei *n* eine natürliche Zahl von 2 bis 5 ist, wobei in m Reaktoren Phosgen aus Kohlenstoffmonoxid und Chlor hergestellt wird, wobei *m* eine natürliche Zahl im Bereich von 1 bis *n -* 1 ist, während in mindestens einem Reaktor die Reinigungsschritte a) bis e) und optional f) durchgeführt werden.

In einer **vierzehnten Ausführungsform** der Erfindung, die eine besondere Ausgestaltung der neunten, zehnten, elften, zwölften und dreizehnten Ausführungsform ist, wird nach Schritt e) oder, sofern durchgeführt, nach Schritt f), die Aktivkohle entfernt und durch frische Aktivkohle ersetzt.

In einer **fünfzehnten Ausführungsform** der Erfindung, die mit allen anderen Ausführungsformen kombinierbar ist, wird als Inertgas in allen Schritten, in denen der Einsatz eines Inertgases vorgesehen ist, Stickstoff eingesetzt.

Die zuvor kurz geschilderten Ausführungsformen und weitere mögliche Ausgestaltungen der Erfindung werden im Folgenden näher erläutert. Verschiedene Ausführungsformen sind, sofern sich aus dem Kontext für den Fachmann nicht eindeutig das Gegenteil ergibt, beliebig miteinander kombinierbar.

Vor der Durchführung des erfindungsgemäßen Verfahrens ist selbstverständlich die Zufuhr der in den zu reinigenden Apparat normalerweise eingeleiteten Ausgangsmaterialien (im Falle eines Phosgenherstellungsreaktors also Kohlenstoffmonoxid und Chlor, im Falle eines Reaktors zur Phosgenierung von Isocyanaten also Amin und Phosgen, usw.) zu unterbrechen und erforderlichenfalls ein vorhandener Flüssigkeitsstand (z. B. an Reaktionslösung) abzulassen.

In **Schritt a**) des erfindungsgemäßen Verfahrens wird dann der zu reinigende Apparat für die weiteren Schritte vorbereitet. Zu diesem Zweck werden eine *Einrichtung zur Gaszufuhr und eine Möglichkeit zur Gasabfuhr bereitgestellt.* Dies kann beispielsweise geschehen, indem entsprechende Leitungen an vorhandene, im regulären Betrieb verschlossene, Flansche angebracht werden oder indem fest installierte Gaszufuhr- und Gasabfuhrleitungen, die im regulären Betrieb des zu reinigenden Apparats durch Einrichtungen wie Ventile verschlossen sind, geöffnet werden. Selbstverständlich umfasst die erfindungsgemäße Vorgehensweise der *"Bereitstellung einer Einrichtung zur Gasabfuhr aus dem und zur Gaszufuhr in den zu reinigenden Apparat"* auch Ausgestaltungen, in denen zwei oder mehr Gaszuleitungen und/oder zwei oder mehr Gasableitungen bereitgestellt werden.

Zusätzlich werden alle darüber hinaus noch vorhandenen Leitungen (wie Leitungen für die Zufuhr und Abfuhr der im regulären Betrieb in dem zu reinigenden Apparat vorhandenen Stoffe) abgesperrt. Der Begriff *"Absperren"* umfasst in diesem Zusammenhang sowohl das Schließen von Leitungen durch entsprechende Einrichtungen wie Ventile als auch das komplette Abtrennen von Leitungen und Verschließen der so entstandenen Öffnungen.

In **Schritt b**) wird nun ein heißer Inertgasstrom durch den zu reinigenden Apparat geleitet. Als inertes Gas eignet sich insbesondere Stickstoff, Kohlenstoffdioxid oder ein Edelgas wie Argon oder Helium. Bevorzugt wird Stickstoff eingesetzt. Der heiße Inertgasstrom wird durch die in Schritt a) bereitgestellte Einrichtung zur Gaszufuhr eingeleitet und durch die in Schritt a) bereitgestellte Einrichtung zur Gasabfuhr abgeleitet. Dieser Schritt dient dazu, den Hauptteil des in dem zu reinigenden Apparat vorhandenen Phosgens auszutreiben. Die Einrichtung zur Gasabfuhr ist vorzugsweise mit einer Abgasaufarbeitung umfassend eine Vorrichtung zur Phosgenzersetzung (insbesondere durch katalytische Zersetzung an Aktivkohle in Gegenwart von Wasser) verbunden.

Das heiße Inertgas hat eine Temperatur im Bereich von 100 °C bis 500 °C, bevorzugt im Bereich von 120 °C bis 400 °C, besonders bevorzugt im Bereich von 150 °C bis 350 °C und ganz besonders bevorzugt im Bereich von 250 °C bis 300 °C. Durch die hohe Temperatur wird bewirkt, dass in dem zu reinigenden Apparat gegebenenfalls vorhandenes flüssiges Phosgen verdampft und gasförmig ausgetragen wird. Die Temperatur des Inertgasstroms wird zweckmäßigerweise online mit dem Fachmann bekannten Temperaturfühlern gemessen.

In einer bevorzugten Ausführungsform wird Schritt b) bei vermindertem Druck (unterhalb des Umgebungsdrucks) durchgeführt, was ebenfalls das Austreiben von Phosgen erleichtert. Bevorzugt liegt der Druck im Bereich von 10 mbar bis 1000 mbar, besonders bevorzugt im Bereich von 100 mbar bis 990 mbar, ganz besonders bevorzugt im Bereich von 500 mbar bis 980 mbar. Hierzu wird eine Vakuum erzeugende Pumpe oder, in Druckbereichen ab 500 mbar, ein Ventilator eingesetzt.

Bevorzugt wird der Phosgengehalt (Volumenanteil an Phosgen) des Inertgasstromes, der aus dem zu reinigenden Apparat durch die Gasabführeinrichtung austritt, gemessen. Dies geschieht entweder kontinuierlich oder in Intervallen. In letzterem Fall wird mindestens 1-mal alle 300 h gemessen. Bevorzugt wird in Intervallen im Bereich von 50 h bis 300 h, besonders bevorzugt von 60 h bis 200 h, ganz besonders bevorzugt von 70 h bis 150 h und außerordentlich ganz besonders bevorzugt von 80 h bis 120 h gemessen. Diese Messung kann grundsätzlich nach allen dem Fachmann bekannten Phosgennachweisverfahren erfolgen, die üblicherweise im Rahmen insignifikanter Messungenauigkeiten vergleichbare Resultate liefern. Im Zweifelsfall ist für die Zwecke der vorliegenden Erfindung der per Farbumschlag infolge der Reaktion von Phosgen mit Ethylanilin und Dimethylaminobenzaldehyd im Temperaturbereich von 10 °C bis 30 °C bestimmte Wert maßgeblich. Zu diesem Zweck bietet beispielsweise die Firma Dräger geeignete Messeinrichtungen (wie das "Dräger-Prüfröhrchen" "CH19 401") an. Insbesondere sobald der gemessene Phosgengehalt auf einen Wert von kleiner oder gleich 1 ppm gesunken ist, kann mit dem nächsten Schritt begonnen werden.

In **Schritt c)** des erfindungsgemäßen Verfahrens wird der heiße Inertgasstrom aus Schritt b) abgestellt und der zu reinigende Apparat abgekühlt, und zwar auf eine (zuvor festgelegte) Temperatur im Bereich von 0 °C bis 70 °C, bevorzugt im Bereich von 10 °C bis 60 °C, besonders bevorzugt im Bereich von 20 °C bis 50 °C, ganz besonders bevorzugt im Bereich von 30 °C bis 40 °C. Diese Abkühlung erfolgt durch Durchleiten eines vergleichsweise kalten Inertgasstroms, nämlich durch Durchleiten eines Inertgasstroms, der eine Temperatur gleich oder unterhalb der zuvor festgelegten Temperatur aufweist. Als Inertgase eignen sich die gleichen wie für Schritt b). Der vergleichsweise kalte Inertgasstrom wird durch die in Schritt a) bereitgestellte Einrichtung zur Gaszufuhr eingeleitet und durch die in Schritt a) bereitgestellte Einrichtung zur Gasabfuhr abgeleitet. Die Abkühlung kann auch realisiert werden, indem der Inertgasstrom aus Schritt b) nicht mehr (oder nicht mehr in dem Ausmaß wie in Schritt b)) beheizt wird, sodass seine Temperatur sukzessive fällt. In dieser Ausführungsform findet ein fließender Übergang von Schritt b) nach Schritt c) statt, und das *"Abstellen"* des heißen Intertgasstromes aus Schritt b) meint dann einfach eine Erniedrigung seiner Temperatur auf einen kleineren Wert als in Schritt b) minimal gefordert, also auf einen Wert von unter 100 °C (bzw., in bevorzugten Ausführungsformen, von unter 120 °C bzw. von unter 150 °C bzw. von unter 250 °C). Der Erfolg der Abkühlung des zu reinigenden Apparats wird zweckmäßigerweise durch Messung der Temperatur des aus der Einrichtung zur Gasabfuhr austretenden Inertgasstroms überprüft.

Der vergleichsweise kalte Inertgasstrom aus Schritt c) wird bevorzugt auch nach erfolgter Abkühlung des zu reinigenden Apparats beibehalten.

In **Schritt d)** des erfindungsgemäßen Verfahrens wird gasförmiger Ammoniak aus einer Ammoniakgasquelle durch den zu reinigenden Apparat geleitet. Die Ammoniakgasquelle kann eine beliebige, dem Fachmann grundsätzlich bekannte, Einrichtung zur Bereitstellung gasförmigen Ammoniaks sein. Bevorzugt wird ein Druckbehälter eingesetzt, in dem trockener Ammoniak unter gegenüber Umgebungsdruck erhöhtem Druck (bevorzugt bei einem Druck im Bereich von 5 bar_{(ü)} bis 15 bar_{(ü)}) verflüssigt vorliegt. In dieser Ausführungsform wird der flüssige Ammoniak aus dem Druckbehälter in die in Schritt a) bereitgestellte Einrichtung zur Gaszufuhr eingeleitet, wo der Ammoniak infolge Entspannung spontan verdampft. Die Ableitung des Ammoniaks oder Ammoniak-haltigen Gasgemisches aus dem zu reinigenden Apparat erfolgt durch die in Schritt a) bereitgestellte Einrichtung zur Gasabfuhr.

In bevorzugter Ausgestaltung geschieht die Einspeisung des Ammoniaks, indem der Ammoniakgasstrom in den (in dieser Ausführungsform aufrechterhaltenen) Inertgasstrom aus Schritt c) eingespeist wird. Die damit einhergehende Verdünnung des Ammoniaks hat den Vorteil, dass die Bildung lokaler Hotspots vermieden wird. Hierbei wird ein Ammoniak-haltiges Gasgemisch (Gemisch aus Ammoniak und Inertgas) erhalten. Der Mengenstrom des gasförmigen Ammoniaks wird dabei so gewählt, dass die Temperatur in dem aus dem zu reinigenden Apparat abgeführten Ammoniak-haltigen Gasgemisch kontinuierlich ansteigt. Dieser Temperaturanstieg ist auf die Exothermie der Reaktion von Phosgen mit Ammoniak zurückzuführen; die Konzentration des eingespeisten Ammoniaks im Inertgasstrom muss hinreichend hoch sein, um ein Ablaufen der Zersetzungsreaktion mit hinreichender Geschwindigkeit und hinreichendem Temperaturanstieg zu gewährleisten. Der Volumenanteil des in Schritt d) eingespeisten gasförmigen Ammoniaks beträgt daher bevorzugt von 0,01 % bis 0,1 %, bezogen auf das Gesamtvolumen aus Inertgasstrom und eingespeistem Ammoniak. Der erforderliche Mengenstrom des in Schritt d) eingespeisten gasförmigen Ammoniaks kann vom Fachmann durch einfache Vorversuche leicht ermittelt werden. Für den Fall, dass der zu reinigende Apparat ein mit Aktivkohle bestückter Reaktor, beispielsweise ein Reaktor zur Herstellung von Phosgen oder ein Reaktor zur Absorption oder Zersetzung von Phosgen in Phosgen-haltigen Gasströmen, ist, werden bevorzugt 3 L/h bis 30 L/h Ammoniak pro Tonne Aktivkohle im zu reinigenden Apparat eingesetzt. In diesen Fällen dient das erfindungsgemäße Verfahren insbesondere dazu, einen solchen Reaktor vor dem Austausch der im Reaktor vorhandenen Aktivkohle durch frische Aktivkohle von Phosgenresten (die auch in der Aktivkohle selbst sein können) zu befreien. Dabei bezieht sich die Volumenstromangabe "L/h" auf das unter den gegebenen Bedingungen von Druck und Temperatur tatsächlich vorliegende Volumen des gasförmigen Ammoniaks, welches mit dem Fachmann bekannten Durchflussmessgeräten, wie z. B. einem Rotameter, leicht bestimmt werden kann.

Durch die exotherme Zersetzungsreaktion in Schritt d) steigt die Temperatur zunächst kontinuierlich an. Nach Zersetzung des vorhanden Phosgens fällt die Temperatur wieder; es wird also ein Temperaturmaximum durchlaufen. Dieses liegt im Bereich von 30 °C bis 120 °C, bevorzugt im Bereich von 50 °C bis 110 °C, besonders bevorzugt im Bereich von 60 °C bis 100 °C und ganz besonders bevorzugt im Bereich von 70 °C bis 90 °C. Sobald dieses Temperaturmaximum durchlaufen ist, kann die Ammoniakgaszufuhr abgestellt werden. Zur Erhöhung der Sicherheit kann es zweckmäßig sein, die Ammoniakgaszufuhr in Schritt d) noch für einen Zeitraum von 1 h bis 8 h nach Erreichen des Temperaturmaximums aufrechtzuerhalten.

In **Schritt e)** wird die Ammoniakgasquelle von dem zu reinigenden Apparat abgetrennt und ein Intertgasstrom durch den zu reinigenden Apparat geleitet. In der oben bereits erwähnten bevorzugten Ausführungsform geschieht dies einfach durch weitere Aufrechterhaltung des Inertgasstromes aus Schritt c). Die Intertgaszufuhr aus Schritt e) wird bevorzugt für einen Zeitraum von 1 Minute bis 2 h aufrechterhalten.

In vielen Fällen ist der zu reinigende Apparat nach der Durchführung von Schritt e) bereits hinreichend von Phosgen befreit, um, beispielsweise für Wartungs-, Instandsetzungs- und Reinigungsarbeiten, geöffnet zu werden. In Fällen besonders hartnäckiger Phosgenkontamination kann es erforderlich sein, einen weiteren Reinigungsschritt anzuschließen.

In diesem optionalen **Schritt f)** wird der zu reinigende Apparate mit einem wässrigen Strom gespült. Zu diesem Zweck wird zunächst eine Flüssigkeitszuleitung in den und eine Flüssigkeitsableitung aus dem zu reinigenden Apparat bereitgestellt.

Dies kann beispielsweise geschehen, indem entsprechende Leitungen an vorhandene, im regulären Betrieb verschlossene, Flansche angebracht werden oder indem fest installierte Flüssigkeitszuleitungen und Flüssigkeitsableitungen, die im regulären Betrieb des zu reinigenden Apparats durch Einrichtungen wie Ventile verschlossen sind oder für andere Zwecke genutzt werden, geöffnet werden. Selbstverständlich umfasst die erfindungsgemäße Vorgehensweise der *"Bereitstellung einer Flüssigkeitszuleitung in den und einer Flüssigkeitsableitung aus dem zu reinigenden Apparat"* auch Ausgestaltungen, in denen zwei oder mehr Flüssigkeitszuleitungen und/oder zwei oder mehr Flüssigkeitsableitungen bereitgestellt werden.

Als *"wässriger Strom"* in Schritt d) eignen sich Wasserqualitäten wie destilliertes Wasser, vollentsalztes Wasser, Wasser aus dem zur Verfügung stehenden Leitungsnetz, Dampfkondensat (in Wärmeaustauschern kondensierter Wasserdampf) sowie wässrige Prozessströme (wässrige Ströme, die in der chemischen Produktion anfallen, sofern sie nicht sauer sind, weil dies Korrosion begünstigen würde).

Wenn der Schritt d) durchgeführt wird, so wird das Durchleiten des wässrigen Stroms insbesondere mindestens so lange aufrechterhalten, bis der pH-Wert des aus dem Apparat austretenden wässrigen Stroms im Bereich von 7,0 bis 10,0, bevorzugt von 7,0 bis 9,0, besonders bevorzugt von 7,0 bis 8,0, liegt. Im Rahmen der vorliegenden Erfindung beziehen sich pH-Werte auf eine Temperatur von 30 °C. Hierdurch wird bewirkt, dass der Apparat keinem sauren, korrosiven Milieu ausgesetzt wird.

Das erfindungsgemäße Verfahren eignet sich in besonderer Weise zur Reinigung eines mit Aktivkohle befüllten Reaktors zur Herstellung von Phosgen aus Kohlenstoffmonoxid und Chlor. Solche Reaktoren werden insbesondere als Bestandteil von Produktionsanlagen zur Herstellung chemischer Produkte durch Umsetzung von mit Phosgen reaktionsfähigen Ausgangsmaterialien mit Phosgen eingesetzt. Als mit Phosgen reaktionsfähige Ausgangsmaterialien eignen sich insbesondere Verbindungen mit zwei oder mehr phenolischen Hydroxygruppen (unter Bildung von *Polycarbonaten*) und Verbindungen mit zwei oder mehr primären Amingruppen (unter Bildung von *Polyisocyanaten*).

In einer bevorzugten Ausführungsform der Erfindung verfügt die Produktionsanlage zur Herstellung des chemischen Produkts über *n* unabhängig voneinander regelbare, mit Aktivkohle befüllte Reaktoren zur Herstellung von Phosgen aus Kohlenstoffmonoxid und Chlor, wobei *n* eine natürliche Zahl von 2 bis 5 ist, wobei in m Reaktoren Phosgen aus Kohlenstoffmonoxid und Chlor hergestellt wird, wobei *m* eine natürliche Zahl im Bereich von 1 bis *n* - 1 ist, während in mindestens einem Reaktor die Reinigungsschritte a) bis e) und optional f) durchgeführt werden. Auf diese Weise kann in einem der Phosgenherstellungsreaktoren der Aktivkohle-Katalysator gewechselt werden, ohne den Betrieb der anderen Phosgenherstellungsreaktoren unterbrechen zu müssen.

Das erfindungsgemäße Verfahren ermöglicht demnach eine besonders vorteilhafte Ausgestaltung des Katalysatorwechsels in einem Phosgenherstellungsreaktor. Ein solcher Katalysatorwechsel wird durchgeführt, indem nach Schritt e) oder, sofern durchgeführt, nach Schritt f) in einem **Schritt g)** die Aktivkohle entfernt und durch frische Aktivkohle ersetzt wird.

Insgesamt zeichnet sich das erfindungsgemäße Verfahren mindestens durch die folgenden Vorteile aus:
(i) Einfache Kontrolle des Verlaufs der Phosgen-Zersetzungsreaktion über die Temperatur.
(ii) Da der Zeitpunkt für die Zersetzung von Phosgen sofort erkannt wird, können die Reparatur- bzw. andere Instandsetzungsmaßnahmen sofort aufgenommen werden, und zeitliche Verzögerungen, die Produktverlust zur Folge hätten, werden vermieden.
(iii) Die Phosgenzersetzungsreaktion wird in neutralem bis alkalischen Milieu durchgeführt, was das metallische Equipment vor einem möglichen Säureangriff schützt.
(iv) In der bevorzugten Ausführungsform unter Beibehaltung des Inertgasstroms in Schritt d) wird infolge der hierdurch bewirkten Verdünnung des Ammoniaks mit Stickstoff die Bildung von "Hotspots" ("Durchgehen" der Reaktion) vermieden. Die Verdünnung führt zu einer gleichmäßigen Verteilung von Ammoniak und damit zu einem gleichmäßigen Anstieg der exothermen Reaktion.

### Beispiele:

### Beispiel 1 (Vergleichsbeispiel): Aktivkohlewechsel in einem Phosgengenerator ohne Zuhilfenahme von Ammoniak:

Ein Phosgengenerator mit einem Innenvolumen von 12 m³ wurde mit einer Stickstoffzufuhr versehen und an das Abgassystem der Produktionsanlage angeschlossen. Die übrigen Zu- und Ableitungen des Phosgengenerators wurden geschlossen. Nun wurde durch die Stickstoffzufuhr 120 Nm³/h Stickstoff einer Temperatur von 280 °C unter Beibehaltung der Mantelbeheizung des Phosgengenerators für 21 Tage durch den Phosgengenerator durchgeleitet. Dabei wurde mittels eines im Abgassystem der Produktionsanlage installierten Ventilators der Druck am Ausgang des Phosgengenerators auf 980 mbar (absolut) abgesenkt. Nach diesem Vorgang wurde bei Normaldruck im Ausgang des Apparates ein Phosgengehalt < 1 ppm mittels "Dräger-Prüfröhrchen" CH19 401 gemessen. Nun wurde die Mantelbeheizung geschlossen und die Stickstoffzufuhr abgestellt. Zuletzt wurde der Phosgengenerator mit 1000 L/h Dampfkondensat gespült. Zu Beginn hatte das austretende Spülkondensat einen pH-Wert von 2. Nach zwei Tagen Spülen hatte sich graduell ein pH-Wert von 6,7 eingestellt, und das Spülen wurde beendet. Nach dem Ablassen des Spülkondensates wurde die Aktivkohle aus dem Phosgengenerator innerhalb eines Tages abgesaugt. Der ganze Vorgang dauert 23 Tage. Bei der Inspektion des Phosgengenerators wurde ein leichter Metallabtrag durch Säureangriff festgestellt.

### Beispiel 2 (erfindungsgemäß): Aktivkohlewechsel in einem Phosgengenerator unter Verwendung von gasförmigem Ammoniak:

Ein Phosgengenerator mit einem Innenvolumen von 12 m³ wurde mit einer Stickstoffzufuhr versehen und an das Abgassystem der Produktionsanlage angeschlossen. Die übrigen Zu- und Ableitungen des Phosgengenerators wurden geschlossen **(Schritt a)).** Nun wurde durch die Stickstoffzufuhr 120 Nm³/h Stickstoff einer Temperatur von 280 °C unter Beibehaltung der Mantelbeheizung des Phosgengenerators für 7 Tage durch den Phosgengenerator durchgeleitet. Dabei wurde mittels eines im Abgassystem der Produktionsanlage installierten Ventilators der Druck am Ausgang des Phosgengenerators auf 980 mbar (absolut) abgesenkt. Nach diesem Vorgang wurde bei Normaldruck im Ausgang des Apparates ein Phosgengehalt < 1 ppm mittels "Dräger-Prüfröhrchen" CH19 401 gemessen **(Schritt b)).** Nun wurde die Mantelbeheizung geschlossen. Danach wurde der Apparat mit Stickstoff einer Temperatur von 25 °C für 1 Tag gespült und hatte anschließend im Gasaustritt eine Temperatur von 30 °C (**Schritt c**)). Der kalte Stickstoff lief mit 120 Nm³/h weiter, und über ein Nadelventil (Eckventilregulierer von Firma Hofer) wurden 50 L/h Ammoniakgas, das als trockener Ammoniak einem Druckbehälter von 10 bar_{(ü)} entnommen wurde, in den kalten Stickstoff-Strom eindosiert **(Schritt d)).** Die Temperatur im Austritt des Phosgenvereinigers stieg innerhalb von 10 Stunden von 30 °C auf 80 °C. In der darauffolgenden Stunde blieb die Temperatur konstant und fiel danach ab. Dann wurde die Zufuhr von Ammoniakgas abgestellt, und es wurde für weitere 2 Stunden mit kaltem Stickstoff (120 Nm³/h, 25 °C) gespült (**Schritt e**)). Nun wurde die Stickstoffzufuhr abgestellt. Zuletzt wurde der Phosgengenerator mit 1000 L/h Dampfkondensat einer Temperatur von 28 °C gespült, bis sich ein pH-Wert von 7,8 eingestellt hatte, was einen Tag in Anspruch nahm (**Schritt f**)). Das Waschwasser wurde, so wie es anfiel, kontinuierlich über einen Schwerstoffabscheider der Abwasserbehandlung einer Kläranlage zugeführt. Nach dem Ablassen des Waschwassers wurde die Aktivkohle aus dem Phosgengenerator innerhalb eines Tages abgesaugt. Die abgesaugte Aktivkohle wurde in einer Verbrennungsanlage verbrannt. Der ganze Vorgang dauert 11 Tage. Bei der Inspektion des Phosgengenerators wurde kein Metallabtrag festgestellt.

## Patentansprüche

1. Verfahren zur Reinigung eines Phosgen-führenden Apparats, umfassend die Schritte:
a) Bereitstellung einer Einrichtung zur Gasabfuhr aus dem zu reinigenden Apparat und einer Einrichtung zur Gaszufuhr in den zu reinigenden Apparat und Absperren aller gegebenenfalls darüber hinaus vorhandenen mit dem zu reinigenden Apparat verbundenen Leitungen;
b) Durchleiten eines Inertgases einer Temperatur im Bereich von 100 °C bis 500 °C durch den zu reinigenden Apparat;
c) Abstellen des Inertgasstroms aus Schritt b), gefolgt von Abkühlen des zu reinigenden Apparates auf eine zuvor festgelegte, in der Einrichtung zur Gasabfuhr gemessene, Temperatur im Bereich von 0 °C bis 70 °C durch Durchleiten eines Inertgases einer Temperatur gleich oder unterhalb der zuvor festgelegten Temperatur,
wobei ferner der Inertgasstrom aus Schritt c) auch nach erfolgter Abkühlung des zu reinigenden Apparates weiter aufrecht erhalten werden kann;
d) sobald das Abkühlen des zu reinigenden Apparates in Schritt c) erfolgt ist, Durchleiten von gasförmigem Ammoniak aus einer Ammoniakgasquelle unter Erhalt eines Ammoniak-haltigen Gasgemisches, wobei der Mengenstrom des gasförmigen Ammoniaks so gewählt wird, dass die Temperatur in dem aus dem zu reinigenden Apparat abgeführten Gasgemisch kontinuierlich ansteigt und nach Erreichen eines Maximums wieder abfällt, wobei das Maximum der Temperatur im Bereich von 30 °C bis 120 °C, liegt;
e) frühestens nach Erreichen des Temperaturmaximums in Schritt d), Abtrennen der Ammoniakgasquelle von dem zu reinigenden Apparat, gefolgt von Durchleiten eines Intergases durch den zu reinigenden Apparat;
f) optional, nach Abstellung des Inertgasstromes, Bereitstellung einer Flüssigkeitszuleitung in den und einer Flüssigkeitsableitung aus dem zu reinigenden Apparat und Durchleiten eines wässrigen Stroms.

2. Verfahren gemäß Anspruch 1, bei welchem Schritt b) bei einem Druck unterhalb von Umgebungsdruck durchgeführt wird.

3. Verfahren gemäß Anspruch 2, bei welchem der Druck in Schritt b) im Bereich von 10 mbar bis 1000 mbar liegt.

4. Verfahren gemäß einem der vorstehenden Ansprüche, bei welchem Schritt b) so lange durchgeführt wird, bis der kontinuierlich oder in Intervallen von bis zu 300 h in dem aus dem zu reinigenden Apparat abgeführten Inertgas per Farbumschlag infolge der Reaktion von Phosgen mit Ethylanilin und Dimethylaminobenzaldehyd im Temperaturbereich von 10 °C bis 30 °C bestimmte, auf das Gesamtvolumen des die mindestens eine Ableitung pro Zeitspanne durchströmenden Gasstroms bezogene, Volumenanteil an Phosgen auf einen Wert von kleiner oder gleich 1 ppm fällt.

5. Verfahren gemäß einem der vorstehenden Ansprüche, bei welchem die zuvor festgelegte, in der Einrichtung zur Gasabfuhr gemessene, Temperatur aus Schritt c) im Bereich von 10 °C bis 60 °C liegt.

6. Verfahren gemäß einem der vorstehenden Ansprüche, bei welchem der Inertgasstrom aus Schritt c) nach erfolgter Abkühlung des zu reinigenden Apparates weiter aufrecht erhalten wird.

7. Verfahren gemäß Anspruch 6, bei welchem das Durchleiten von gasförmigem Ammoniak aus einer Ammoniakgasquelle in Schritt d) durch Einspeisung in den aufrecht erhaltenen Intertgasstrom aus Schritt c) erfolgt.

8. Verfahren gemäß Anspruch 6 oder 7, bei welchem das Durchleiten eines Intergases durch den zu reinigenden Apparat in Schritt e) nur durch Aufrechterhaltung des Inertgasstromes aus Schritt c) bewirkt wird.

9. Verfahren gemäß einem der vorstehenden Ansprüche, bei welchem Schritt f) durchgeführt wird und der wässrige Strom mindestens so lange durch den zu reinigenden Apparat geleitet wird, bis der pH-Wert des aus dem Apparat austretenden wässrigen Stroms im Bereich von 7,0 bis 10,0 liegt.

10. Verfahren gemäß einem der vorstehenden Ansprüche, bei welchem der zu reinigende Apparat (i) ein mit Aktivkohle befüllter Reaktor geeignet zur Herstellung von Phosgen aus Kohlenstoffmonoxid und Chlor oder (ii) ein mit Aktivkohle befüllter Reaktor geeignet zur Absorption oder Zersetzung von Phosgen in Phosgen-haltigen Gasströmen ist, wobei der Mengenstrom an gasförmigem Ammoniak, der in Schritt d) durch den mit Aktivkohle befüllten Reaktor geleitet wird, im Bereich von 3 L/h bis 30 L/h pro Tonne Aktivkohle liegt.

11. Verfahren gemäß einem der vorstehenden Ansprüche, bei welchem der zu reinigende Apparat ein mit Aktivkohle befüllter Reaktor geeignet zur Herstellung von Phosgen aus Kohlenstoffmonoxid und Chlor ist.

12. Verfahren gemäß Anspruch 11, bei welchem der mit Aktivkohle befüllte Reaktor geeignet zur Herstellung von Phosgen aus Kohlenstoffmonoxid und Chlor Bestandteil einer Produktionsanlage zur Herstellung eines chemischen Produkts durch Umsetzung eines mit Phosgen reaktionsfähigen Ausgangsmaterials mit Phosgen ist.

13. Verfahren gemäß Anspruch 12, bei welchem das mit Phosgen reaktionsfähige Ausgangsmaterial eine Verbindung mit zwei oder mehr phenolischen Hydroxygruppen oder eine Verbindung mit zwei oder mehr primären Amingruppen ist.

14. Verfahren gemäß einem der Ansprüche 12 oder 13, bei welchem die Produktionsanlage zur Herstellung eines chemischen Produkts über *n* unabhängig voneinander regelbare, mit Aktivkohle befüllte Reaktoren geeignet zur Herstellung von Phosgen aus Kohlenstoffmonoxid und Chlor verfügt, wobei *n* eine natürliche Zahl von 2 bis 5 ist, wobei in m Reaktoren Phosgen aus Kohlenstoffmonoxid und Chlor hergestellt wird, wobei m eine natürliche Zahl im Bereich von 1 bis *n -* 1 ist, während in mindestens einem Reaktor die Reinigungsschritte a) bis e) und optional f) durchgeführt werden.

15. Verfahren gemäß einem der Ansprüche 10 bis 14, bei welchem nach Schritt e) oder, sofern durchgeführt, nach Schritt f):
g) die Aktivkohle entfernt und durch frische Aktivkohle ersetzt wird.
